(19) Europäisches Patentamt
European Patent Office
Office européen des brevets

(11) Numéro de publication : **0 556 092 A1**

(12) # DEMANDE DE BREVET EUROPEEN

(21) Numéro de dépôt : **93400272.6**

(22) Date de dépôt : **04.02.93**

(51) Int. Cl.$^5$ : **C07D 211/90,** C07D 409/12, C07D 401/12, C07D 405/04, C07D 409/14, C07D 405/14, A61K 31/44, A61K 31/47

(30) Priorité : **05.02.92 FR 9201266**

(43) Date de publication de la demande : **18.08.93 Bulletin 93/33**

(84) Etats contractants désignés : **AT BE CH DE DK ES FR GB GR IE IT LI LU NL PT SE**

(71) Demandeur : **ADIR ET COMPAGNIE**
**1 rue Carle Hébert**
**F-92415 Courbevoie Cédex (FR)**

(72) Inventeur : **Peglion, Jean-Louis**
**5 Allée des Bégonias**
**F-78110 Le Vesinet (FR)**
Inventeur : **Atassi, Ghanem**
**4 rue Joséphine**
**F-92400 Saint-Cloud (FR)**
Inventeur : **Pierre, Alain**
**52 rue de Montval**
**F-78160 Marly Le Roi (FR)**
Inventeur : **Kraus-Berthier, Laurence**
**20 rue Petite des Champarons**
**F-92700 Colombes (FR)**
Inventeur : **Guilbaud, Nicolas**
**24 rue Lefèvre**
**F-75015 Paris (FR)**
Inventeur : **Vilaine, Jean-Paul**
**21 rue des Vallées**
**F-92290 Chatenay Malabry (FR)**

(74) Mandataire : **Reverbori, Marcelle**
**Adir et Compagnie, 1, rue Carle Hébert**
**F-92415 Courbevoie Cédex (FR)**

(54) **Composés de 1,4-dihydropyridine, leur procédé de préparation et les compositions pharmaceutiques les contenant.**

(57) Les composés de 1,4-dihydropyridine de formule I :

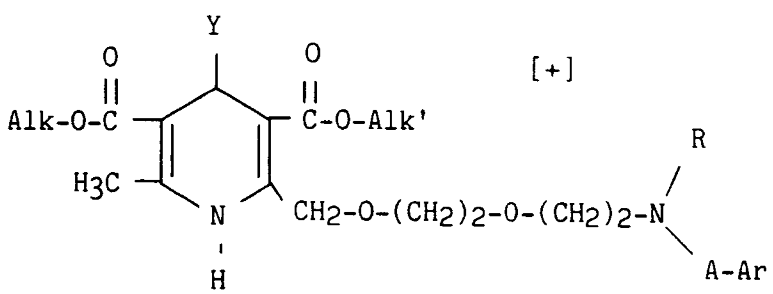

dans laquelle :

— Y représente un radical phényle éventuellement substitué par 1 à 5 substituants identiques ou différents représentant chacun un atome d'halogène, ou un radical alkoxy ou alkylthio ayant chacun de 1 à 4 atomes de carbone, un radical trihalogénométhyle ou un radical méthylène-dioxy ;

— Alk et Alk', identiques ou différents, représentent chacun un radical alkyle de 1 à 5 atomes de carbone, en chaine linéaire ou ramifiée ;

— R représente un atome d'hydrogène, un radical alkyle de 1 à 5 atomes de carbone en chaine linéaire ou ramifiée, ou un radical alkényle ou alkinyle ayant chacun de 3 à 5 atomes de carbone en chaine linéaire ou ramifiée ;

— A représente :
une chaine $-(CH_2)_m-$ dans laquelle m est un nombre entier de 1 à 5, éventuellement substituée par un radical alkyle de 1 à 5 atomes de carbone en chaine droite ou ramifiée, ou par un radical alkényle ou alkinyle ayant de 2 à 5 atomes de carbone en chaine droite ou ramifiée, et simultanément

— Ar représente :
a) un radical phényle éventuellement substitué par un ou plusieurs atomes d'halogène ou radicaux

EP 0 556 092 A1

trifluorométhyle, nitro, cyano, alkoxycarbonyl de 2 à 6 atomes de carbone ou alkylthio de 1 à 5 atomes de carbone,

b) un radical thiényle, pyridyle, naphtyle, quinolyle, isoquinolyle, indolyle, N-méthylindolyle, benzofurazanyle ou benzo-2,1,3-thiadiazolyle,

ou

— l'ensemble -A-Ar représente un radical dibenzo(a,d)cyclohept-5-yle ; sous forme d'isomère dextrogyre.

Les composés de formule I et leurs sels d'addition physiologiquement tolérables sont utilisables en thérapeutique anticancéreuse.

La présente invention a pour objet de nouveaux composés de 1,4-dihydropyridine, leur procédé de préparation et les compositions pharmaceutiques les contenant.

Elle concerne particulièrement les composés de 1,4-dihydropyridine de formule I :

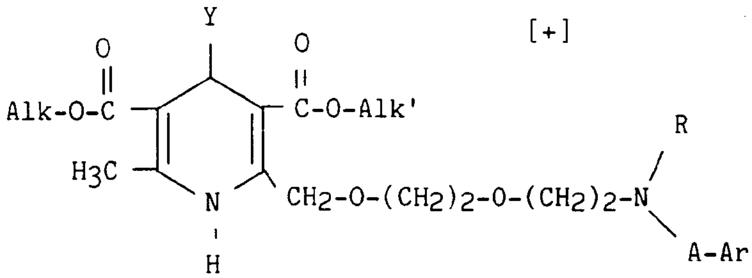

[+]

(I)

dans laquelle :

- Y représente un radical phényle éventuellement substitué par 1 à 5 substituants identiques ou différents représentant chacun un atome d'halogène, tel que par exemple un atome de chlore ou de fluor ou un radical alkoxy ou alkylthio ayant chacun de 1 à 4 atomes de carbone, un radical trihalogénométhyle ou un radical méthylène-dioxy ;
- Alk et Alk', identiques ou différents, représentent chacun un radical alkyle de 1 à 5 atomes de carbone, en chaine linéaire ou ramifiée ;
- R représente un atome d'hydrogène, un radical alkyle de 1 à 5 atomes de carbone en chaine linéaire ou ramifiée, ou un radical alkényle ou alkinyle ayant chacun de 3 à 5 atomes de carbone en chaine linéaire ou ramifiée ;
- A représente :

  une chaine $-(CH_2)_m-$ dans laquelle m est un nombre entier de 1 à 5, éventuellement substituée par un radical alkyle de 1 à 5 atomes de carbone en chaine droite ou ramifiée, ou par un radical alkényle ou alkinyle ayant chacun de 2 à 5 atomes de carbone en chaine droite ou ramifiée,

  et simultanément
- Ar représente :

  a) un radical phényle éventuellement substitué par un ou plusieurs atomes d'halogène ou radicaux trifluorométhyle, nitro, cyano, alkoxycarbonyl de 2 à 6 atomes de carbone ou alkylthio de 1 à 5 atomes de carbone,

  b) un radical thiényle, pyridyle, naphtyle, quinolyle, isoquinolyle, indolyle, N-méthylindolyle, benzofurazanyle ou benzo-2,1,3-thiadiazolyle,

  ou
- l'ensemble -A-Ar représente un radical dibenzo(a,d)cyclohept-5-yle ;

sous forme d'isomère dextrogyre, et leurs sels d'addition physiologiquement tolérables.

Il est connu de l'état antérieur de la technique que les 1,4-dihydropyridines ont principalement des propriétés pharmacologiques au niveau du système cardiovasculaire, par inhibition de l'influx du calcium, transitant par les canaux calciques vers l'intérieur des cellules du muscle lisse vasculaire (bloqueurs des canaux calciques ou anticalciques), et servent ainsi de médicaments pour l'hypertension et l'angine de poitrine.

D'autre part, le brevet US 4.690.935 mentionne que les 1,4-dihydropyridines telles que Nimodipine ou Nifédipine possèdent des propriétés antitumorales, et le brevet EP 221.382 stipule que ces mêmes 1,4-dihydropyridines, en combinaison avec un composé de coordination du platine, permettent le traitement des tumeurs. Toutefois, les 1,4-dihydropyridines objets de ces brevets possèdent, avant tout, de puissantes propriétés anticalciques, les rendant difficilement utilisables en thérapeutique antitumorale, à cause de leurs effets trop prononcés sur le système cardiovasculaire.

Les brevets EP 270.926 et EP 359.377 concernent des 1,4-dihydropyridines utilisables en thérapeutique antitumorale, et n'ayant que peu de propriétés anticalciques, lesquelles 1,4-dihydropyridines sont substituées en position 4 non par un noyau benzénique mais par des radicaux hétérocycliques de type soit pyrazolo [1,5-a] pyrid-3-yle (cf brevet EP 270.926) soit 5,6-dihydro-p-dioxin-2 yle et 3-méthyl 5,6-dihydro 1,4-dithin-2-yle (cf brevet EP 359.377).

Les brevets EP 0.259.206, 0.419.297 et 0.406.502 revendiquent des 1,4-dihydropyridines ayant de fortes propriétés anticalciques conférant aux dites molécules des activités cardiovasculaires puissantes associées à de faibles propriétés de type adjuvant dans le traitement du cancer.

Des recherches intensives dans les Services de la demanderesse ont abouti aux composés objets de la présente invention, lesquels se différencient des composés les plus proches de l'état antérieur de la technique

non seulement par leur structure mais aussi par leur activité spécifique et bénéfique au niveau du phénomène de résistance des cellules tumorales à la chimiothérapie.

En effet, les composés de la présente invention augmentent, de façon très importante, la sensibilité des cellules tumorales aux agents antitumoraux ainsi que la sensibilité des cellules tumorales ayant acquis une résistance à différents agents anticancéreux (ou résistance multidrogue), tout en ne présentant que de faibles propriétés anticalciques, ce qui supprime leur effet pharmacologique hypotenseur et les rend utilisables en thérapeutique anticancéreuse sans entrainer d'effets secondaires gênants.

La présente invention a également pour objet le procédé de préparation des composés de formule I caractérisé en ce que l'on transforme l'isomère dextrogyre de l'amine primaire de formule II :

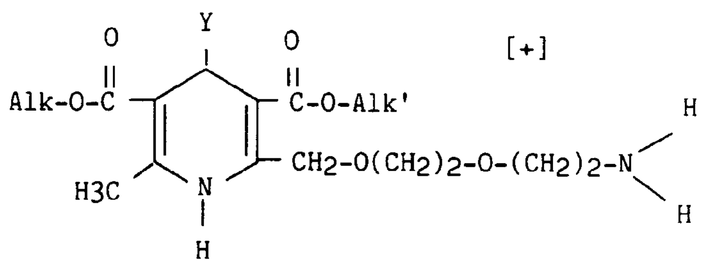

(II)

(dans laquelle Y, Alk et Alk' ont les significations précédemment définies)
en isomère dextrogyre de l'amine secondaire ou tertiaire correspondant de formule I.

Cette transformation est effectuée selon les méthodes classiques utilisées en chimie organique pour obtenir les amines secondaires et tertiaires à partir des amines primaires correspondantes.
Toutefois, il est particulièrement avantageux d'opérer comme ci-après.

Pour obtenir l'isomère dextrogyre de formule I dans laquelle -A-Ar représente le radical dibenzo (a,d) cyclohept-5-yle, c'est à dire pour obtenir les composés répondant plus précisemment à la formule Ia :

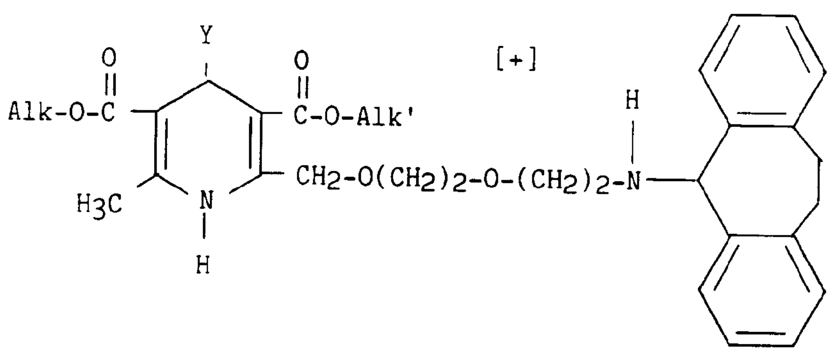

(Ia)

dans laquelle Y, Alk et Alk' ont les significations précédemment définies, la transformation de l'isomère dextrogyre de l'amine primaire de formule II est effectuée au moyen de l'agent de formule III a :

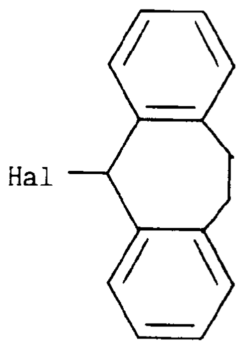

(IIIa)

dans laquelle Hal représente un atome d'halogène.

Il est particulièrement adéquat d'effectuer cette réaction dans un solvant approprié tel que par exemple le cyanure de méthyle ou le diméthylformamide en présence d'un accepteur de l'halogénoacide formé au cours de la réaction - cet accepteur étant un agent alcalin tel que par exemple un carbonate alcalin ou alcalino terreux comme le carbonate de potassium.

Pour obtenir l'isomère dextrogyre des composés I répondant plus précisémment à la formule Ib :

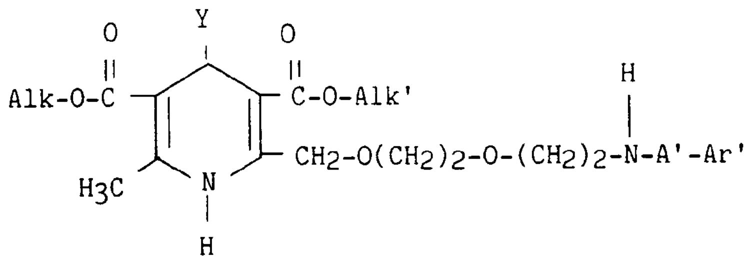

(Ib)

[dans laquelle Y, Alk et Alk' ont les significations précédemment définies et

- A' représente :
  une chaine $(CH_2)_m$ dans laquelle m est un nombre entier de 1 à 5, éventuellement substituée par un radical alkyle ayant de 1 à 5 atomes de carbone en chaine droite ou ramifiée, ou par un radical alkényle ou alkynyle ayant chacun de 2 à 5 atomes de carbone en chaine droite ou ramifiée,
  et simultanément
- Ar' représente :
  a) un radical phényle éventuellement substitué par un ou plusieurs atomes d'halogène ou radicaux trifluorométhyle, nitro, cyano, alkoxycarbonyle de 2 à 6 atomes de carbone, ou alkylthio de 1 à 5 atomes de carbone, ou
  b) un radical thiényle, pyridyle, naphtyle, quinolyle, isoquinolyle, indolyle, N-méthyindolyle, benzofurazanyle ou benzo-2,1,3-thiadiazolyle],

la transformation de l'isomère dextrogyre de l'amine primaire de formule II est effectuée au moyen de l'agent de formule III b :

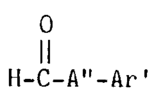

(III b)

dans laquelle :

- Ar' a la signification précédemment définie et
- A" représente une chaîne $(CH_2)_{m-1}$ dans laquelle m est un nombre entier de 1 à 5, éventuellement substituée par un radical alkyle ayant de 1 à 5 atomes de carbone en chaine droite ou ramifiée, ou par un radical alkényle ou alkynyle ayant chacun de 2 à 5 atomes de carbone en chaine droite ou ramifiée,

la transformation s'effectuant avantageusement dans un solvant approprié, comme par exemple l'éthanol, en présence de $NaBH_4$.

Les amines secondaires de formule Ia et Ib précédemment obtenues sont, si on le désire, alcoylées au moyen d'un agent alcoylant approprié pour obtenir les amines tertiaires correspondantes de formule Ic et Id, ci-après :

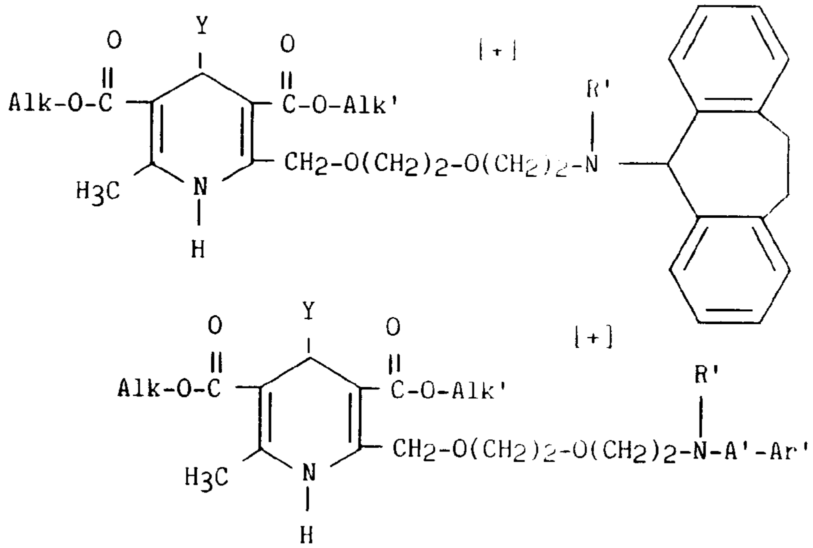

(Ic)

(Id)

dans chacune de ces formules :

- Y, Alk, Alk', A' et Ar' ont les significations précédemment définies, et
- R' représente un radical alkyle de 1 à 5 atomes de carbone en chaine droite ou ramifiée, ou un radical alkényle ou alkinyle ayant chacun de 3 à 5 atomes de carbone en chaine linéaire ou ramifiée (c'est à dire R' a la même signification que R à l'exception d'un atome d'hydrogène).

L'alcoylation des composés Ia et Ib est effectuée de façon particulièrement adéquate :

- soit en condensant les composés Ia et Ib avec un agent de formule IV :

R"-CO-Cl (IV)

dans laquelle :

  - R" représente un radical alkyle ayant de 1 à 4 atomes de carbone en chaine linéaire ou ramifiée, ou un radical alkényle ou alkinyle ayant chacun de 2 à 4 atomes de carbone en chaine linéaire ou ramifiée
  - puis en réduisant l'amide ainsi obtenue, en utilisant par exemple $LiAlH_4$, pour obtenir les composés de formule $Ic_1$ et $Id_1$ :

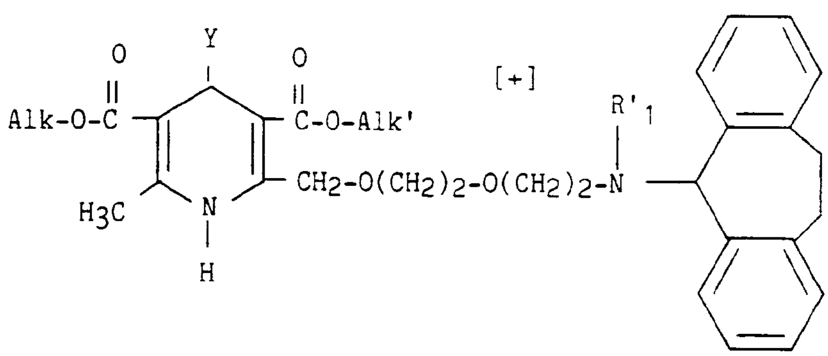

$(Ic_1)$

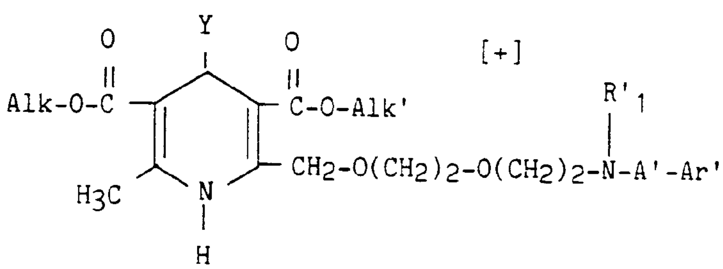

$(Id_1)$

dans lesquelles :

- Y, Alk, Alk', A' et Ar' ont les significations précédemment définies et
- R'$_1$ représente un radical alkyle de 2 à 5 atomes de carbone en chaine droite ou ramifiée ou un radical alkényle ou alkinyle ayant chacun de 3 à 5 atomes de carbone en chaine linéaire ou ramifiée ;
  - soit en faisant réagir les composés Ia et Ib avec acide formique et formol ou phosphate de méthyle à une température comprise entre 60 et 100 °C, pour obtenir les composés de formule $Ic_2$ et $Id_2$ :

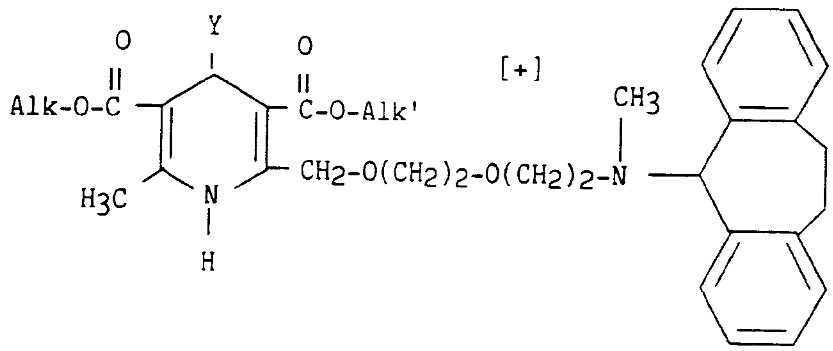
($Ic_2$)

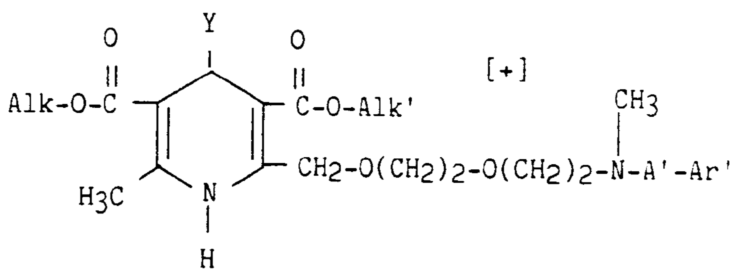
($Id_2$)

dans lesquelles :
- Y, Alk, Alk', A' et Ar' ont les significations précédemment définies.

L'ensemble des composés $Ic_1$, $Id_1$, $Ic_2$, $Id_2$ forme l'ensemble des composés Ic et Id et l'ensemble des composés de formule Ia, Ib, Ic et Id forme l'ensemble des composés de formule I.

Les matières premières de formule II utilisées pour synthétiser les composés I sont des produits connus (cf demande de brevet européen publiée sous le n° 0.419.297).

Les composés de formule I peuvent être transformés en sels d'addition avec les acides, sels qui font, à ce titre, partie de la présente invention. Comme acides utilisables pour la formation de ces sels, on peut citer, par exemple, dans la série minérale les acides chlorhydrique, bromhydrique, sulfurique, nitrique, phosphorique et dans la série organique, les acides acétique, propionique, maléique, fumarique, tartrique, oxalique, benzoïque méthanesulfonique, benzènesulfonique, iséthionique et éthanedioïque monoéthyl ester.

Les composés I peuvent être purifiés par des méthodes physiques ou chimiques classiques.

Les composés de formule I et leurs sels d'addition physiologiquement tolérables possèdent des propriétés pharmacologiques et thérapeutiques intéressantes ; notamment, ils potentialisent l'activité des cytotoxiques et ils augmentent, de façon très importante, la sensibilité des cellules tumorales aux agents antitumoraux -et plus particulièrement la sensibilité des cellules tumorales ayant acquis une résistance à divers agents anticancéreux- tout en ne présentant que de faibles propriétés anticalciques, ce qui permet de les utiliser en thérapeutique anticancéreuse sans entrainer d'effets secondaires génants.

La présente invention a également pour objet les compositions pharmaceutiques contenant, comme principe actif, un composé de formule I ou un de ses sels physiologiquement tolérable, mélangé ou associé à un excipient pharmaceutique approprié.

Les compositions pharmaceutiques ainsi obtenues se présentent généralement sous forme dosée et peuvent contenir de 10 à 500 mg de principe actif.

Elle peuvent revêtir la forme de comprimés, dragées, gélules, suppositoires, solutions injectables ou buvables et être administrées par voie orale, rectale, intraveineuse ou parentérale.

La posologie varie selon l'âge et le poids du patient, la voie d'administration, la nature de la maladie et les traitements associés. Elle s'échelonne généralement de 10 à 500 mg de 1 à 4 fois par jour.

Les exemples suivants illustrent la présente invention ; les points de fusion étant déterminés à la platine chauffante de Kofler, sauf mention contraire.

**Exemple 1**

d-2-{[2-(2-(N-p-nitrobenzylamino)éthoxy) éthoxy] méthyl} 4-(2,3-dichlorophényl) 3-éthoxycarbonyl 5-méthoxycarbonyl 6-méthyl 1,4-dihydropyridine.

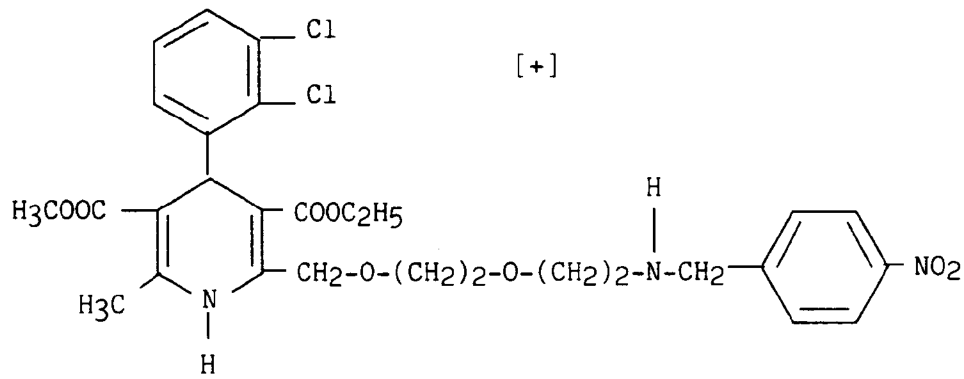

0,01 mole de d-2-{[2-(2-aminoéthoxy)éthoxy]méthyl} 4-(2,3-dichlorophényl) 3-éthoxycarbonyl 5-méthoxycarbonyl 6-méthyl 1,4-dihydropyridine, 0,01 mole de p-nitrobenzaldéhyde et 30 ml d'éthanol sont chauffés à reflux, sous agitation, pendant 2 heures.

Après retour du mélange réactionnel à la température ambiante, on ajoute par portions sous agitation, 0,01 mole de borohydrure de sodium.

L'ensemble est laissé en contact sous agitation pendant une nuit, puis dilué avec 4 volumes d'eau, et extrait à l'acétate d'éthyle.

Le résidu évaporé est soumis à une chromatoflash (éluant : acétate d'éthyle). On obtient ainsi une huile jaune pâle qui est la d-2-{[2-(2-(N-p-nitrobenzylamino)éthoxy)éthoxy]méthyl} 4-(2,3-dichlorophényl) 3-éthoxycarbonyl 5-méthoxycarbonyl 6-méthyl 1,4-dihydropyridine.

0,01 mole de la base ainsi obtenue et 0,01 mole d'acide éthanedioïque monoéthyl ester sont mélangées intimement au bain-marie à 100 °C durant 15 minutes. On observe alors une prise en masse. Le produit obtenu est recristallisé dans l'acétonitrile. On obtient ainsi le monoéthyl oxalate de l'isomère [+] de la 2-{[2-(2-(N-p-nitrobenzylamino) éthoxy) éthoxy] méthyl} 4-(2,3-dichlorophényl) 3-éthoxycarbonyl 5-méthoxycarbonyl 6-méthyl 1,4-dihydropyridine, fondant à 146-148 °C.
Pouvoir rotatoire ($[C]^{21°C}$ = 1 % dans DMSO) :

| λnm | α° |
|---|---|
| 589 | + 24,0 |
| 578 | + 25,7 |
| 546 | + 32,2 |

**Exemples 2 à 27**

En appliquant la méthode de préparation décrite dans l'exemple 1, ont été préparés les composés suivants :

2) d-2-{[2-(2-(N-m-nitrobenzylamino)éthoxy) éthoxy] méthyl} 4-(2,3-dichlorophényl) 3-éthoxycarbonyl 5-méthoxycarbonyl 6-méthyl 1,4-dihydropyridine et son monoéthyl oxalate, PF : 136-138 °C (cyanure de méthyle).

Pouvoir rotatoire ($[C]^{21°C}$ = 1 % dans DMSO) :

| λnm | α° |
|---|---|
| 589 | + 28,8 |
| 578 | + 30,7 |
| 546 | + 38,6 |

3) d-2-{[2-(2-(N-o-nitrobenzylamino)éthoxy) éthoxy] méthyl} 4-(2,3-dichlorophényl) 3-éthoxycarbonyl 5-méthoxycarbonyl 6-méthyl 1,4-dihydropyridine et son monoéthyl oxalate, PF : 130-132 °C (cyanure de méthyle).
Pouvoir rotatoire ($[C]^{21°C}$ = 1 % dans DMSO) :

| λnm | α° |
|---|---|
| 589 | + 27,4 |
| 578 | + 29,3 |
| 546 | + 36,6 |

4) d-2-{[2-(2-(N-p-fluorobenzylamino)éthoxy) éthoxy] méthyl} 4-(2,3-dichlorophényl) 3-éthoxycarbonyl 5-méthoxycarbonyl 6-méthyl 1,4-dihydropyridine et son monoéthyl oxalate PF : 147-149 °C (cyanure de méthyle).
Pouvoir rotatoire ($[C]^{21°C}$ = 1 % dans DMSO) :

| λnm | α° |
|---|---|
| 589 | + 28,7 |
| 578 | + 30,5 |
| 546 | + 38 |

5) d-2-{[2-(2-(N-m-fluorobenzylamino)éthoxy) éthoxy] méthyl} 4-(2,3-dichlorophényl) 3-éthoxycarbonyl 5-méthoxycarbonyl 6-méthyl 1,4-dihydropyridine et son monoéthyl oxalate PF : 151-153 °C (cyanure de méthyle).
Pouvoir rotatoire ($[C]^{21°C}$ = 1 % dans DMSO) :

| λnm | α° |
|---|---|
| 589 | + 29,1 |
| 578 | + 31 |
| 546 | + 38,5 |

6) d-2-{[2-(2-(N-o-fluorobenzylamino)éthoxy) éthoxy] méthyl} 4-(2,3-dichlorophényl) 3-éthoxycarbonyl 5-méthoxycarbonyl 6-méthyl 1,4-dihydropyridine et son monoéthyl oxalate PF : 166-168 °C (cyanure de méthyle).
Pouvoir rotatoire ($[C]^{21°C}$ = 1 % dans DMSO) :

| λnm | α° |
|---|---|
| 589 | + 29,7 |
| 578 | + 31,3 |
| 546 | + 39 |

7) d-2-{[2-(2-(N-p-trifluorométhylbenzylamino)éthoxy) éthoxy] méthyl} 4-(2,3-dichlorophényl) 3-éthoxycarbonyl 5-méthoxycarbonyl 6-méthyl 1,4-dihydropyridine et son monoéthyl oxalate PF : 138-140 °C

(éther isopropylique).
Pouvoir rotatoire ([C]$^{21°C}$ = 1 % dans DMSO) :

| λnm | α° |
|---|---|
| 589 | + 21,0 |
| 578 | + 22,4 |
| 546 | + 28,1 |

8) d-2-{[2-(2-(N-p-cyanobenzylamino)éthoxy) éthoxy] méthyl} 4-(2,3-dichlorophényl) 3-éthoxycarbonyl 5-méthoxycarbonyl 6-méthyl 1,4-dihydropyridine et son monoéthyl oxalate PF : 152-158 °C (cyanure de méthyle).
Pouvoir rotatoire ([C]$^{20,5°C}$ = 1 % dans DMSO) :

| λnm | α° |
|---|---|
| 589 | + 28,5 |
| 578 | + 30,7 |
| 546 | + 38,3 |

9) d-2-{[2-(2-(N-p-méthylthiobenzylamino)éthoxy) éthoxy] méthyl} 4-(2,3-dichlorophényl) 3-éthoxycarbonyl 5-méthoxycarbonyl 6-méthyl 1,4-dihydropyridine et son monoéthyl oxalate PF : 141-143 °C.
Pouvoir rotatoire ([C]$^{21°C}$ = 1 % dans DMSO) :

| λnm | α° |
|---|---|
| 589 | + 28,2 |
| 578 | + 30,2 |
| 546 | + 37,4 |

10) d-2-{[2-(2-(N-p-éthoxycarbonylbenzylamino)éthoxy) éthoxy] méthyl} 4-(2,3-dichlorophényl) 3-éthoxycarbonyl 5-méthoxycarbonyl 6-méthyl 1,4-dihydropyridine et son monoéthyl oxalate PF : 120-123 °C (cyanure de méthyle).
Pouvoir rotatoire ([C]$^{21°C}$ = 1 % dans DMSO) :

| λnm | α° |
|---|---|
| 589 | + 26,8 |
| 578 | + 28,5 |
| 546 | + 35,4 |

11) d-2-{[2-(2-(N-napht-1-ylméthylamino)éthoxy) éthoxy] méthyl} 4-(2,3-dichlorophényl) 3-éthoxycarbonyl 5-méthoxycarbonyl 6-méthyl 1,4-dihydropyridine et son monoéthyl oxalate PF : 140-142 °C (cyanure de méthyle).
Pouvoir rotatoire ([C]$^{21°C}$ = 1 % dans DMSO) :

| λnm | α° |
|---|---|
| 589 | + 28,6 |
| 578 | + 30,5 |
| 546 | + 37,8 |

12) d-2-{[2-(2-(N-napht-2-ylméthylamino)éthoxy) éthoxy] méthyl} 4-(2,3-dichlorophényl) 3-éthoxycarbonyl 5-méthoxycarbonyl 6-méthyl 1,4-dihydropyridine et son monoéthyl oxalate PF : 150-152 °C (cyanure de méthyle).
Pouvoir rotatoire ([C]$^{21°C}$ = 1 % dans DMSO) :

| λnm | α° |
|---|---|
| 589 | + 28,7 |
| 578 | + 30,7 |
| 546 | + 38,1 |

13) d-2-{[2-(2-(N-thién-2-ylméthylamino)éthoxy) éthoxy] méthyl} 4-(2,3-dichlorophényl) 3-éthoxycarbonyl 5-méthoxycarbonyl 6-méthyl 1,4-dihydropyridine et son monoéthyl oxalate PF : 154-156 °C (cyanure de méthyle).
Pouvoir rotatoire ([C]$^{21°C}$ = 1 % dans DMSO) :

| λnm | α° |
|---|---|
| 589 | + 30,6 |
| 578 | + 32,8 |
| 546 | + 40,7 |

14) d-2-{[2-(2-(N-thién-3-ylméthylamino)éthoxy) éthoxy] méthyl} 4-(2,3-dichlorophényl) 3-éthoxycarbonyl 5-méthoxycarbonyl 6-méthyl 1,4-dihydropyridine et son monoéthyl oxalate PF : 151-153 °C (cyanure de méthyle).
Pouvoir rotatoire ([C]$^{21°C}$ = 1 % dans DMSO) :

| λnm | α° |
|---|---|
| 589 | + 30,5 |
| 578 | + 32,6 |
| 546 | + 40,5 |

15) d-2-{[2-(2-(N-pyrid-4-ylméthylamino)éthoxy) éthoxy] méthyl} 4-(2,3-dichlorophényl) 3-éthoxycarbonyl 5-méthoxycarbonyl 6-méthyl 1,4-dihydropyridine et son monoéthyl oxalate PF : 138-140 °C (cyanure de méthyle).
Pouvoir rotatoire ([C]$^{21°C}$ = 1 % dans DMSO) :

| λnm | α° |
|---|---|
| 589 | + 29,6 |
| 578 | + 31,5 |
| 546 | + 37 |

16) d-2-{[2-(2-(N-pyrid-2-ylméthylamino)éthoxy) éthoxy] méthyl} 4-(2,3-dichlorophényl) 3-éthoxycarbonyl 5-méthoxycarbonyl 6-méthyl 1,4-dihydropyridine et son monoéthyl oxalate PF : 124-126 °C (cyanure de méthyle).
Pouvoir rotatoire ([C]$^{21°C}$ = 1 % dans DMSO) :

| λnm | α° |
|---|---|
| 589 | + 32,4 |
| 578 | + 34,6 |
| 546 | + 42,3 |

17) d-2-{[2-(2-(N-pyrid-3-ylméthylamino)éthoxy) éthoxy] méthyl} 4-(2,3-dichlorophényl) 3-éthoxycarbonyl 5-méthoxycarbonyl 6-méthyl 1,4-dihydropyridine et son monoéthyl oxalate PF : 164-166 °C (cyanure de méthyle).
Pouvoir rotatoire ([C]$^{21°C}$ = 1 % dans DMSO) :

| λnm | α° |
|---|---|
| 589 | + 32,3 |
| 578 | + 34,4 |
| 546 | + 42,0 |

18) d-2-{[2-(2-(N-quinol-4-ylméthylamino)éthoxy) éthoxy] méthyl} 4-(2,3-dichlorophényl) 3-éthoxycarbonyl 5-méthoxycarbonyl 6-méthyl 1,4-dihydropyridine et son monoéthyl oxalate PF : 154-156 °C (cyanure de méthyle).
Pouvoir rotatoire ([C]$^{21°C}$ = 1 % dans DMSO) :

| λnm | α° |
|---|---|
| 589 | + 29,2 |
| 578 | + 31,1 |
| 546 | + 38,1 |

19) d-2-{[2-(2-(N-isoquinol-1-yl méthylamino)éthoxy) éthoxy] méthyl} 4-(2,3-dichlorophényl) 3-éthoxycarbonyl 5-méthoxycarbonyl 6-méthyl 1,4-dihydropyridine et son monoéthyl oxalate PF : 126-128 °C.
Pouvoir rotatoire ([C]$^{23°C}$ = 1 % dans DMSO) :

| λnm | α° |
|---|---|
| 589 | + 28,3 |
| 578 | + 30,2 |
| 546 | + 37,6 |

20) d-2-{2-[[2-(2-(N-pyrid-2-yl)éthylamino)éthoxy] éthoxy] méthyl} 4-(2,3-dichlorophényl) 3-éthoxycarbonyl 5-méthoxycarbonyl 6-méthyl 1,4-dihydropyridine et son dichlorhydrate sous forme de lyophilisat.
Pouvoir rotatoire ([C]$^{21°C}$ = 1 % dans DMSO) :

| λnm | α° |
|---|---|
| 589 | + 31,03 |
| 578 | + 33,2 |
| 546 | + 41,2 |

21) d-2-{[2-(2-[N-(N-méthylindol-3-yl) méthylamino]éthoxy) éthoxy] méthyl} 4-(2,3-dichlorophényl) 3-éthoxycarbonyl 5-méthoxycarbonyl 6-méthyl 1,4-dihydropyridine et son monoéthyl oxalate PF : 140-142 °C ($CH_3CN$).
22) d-2-{[2-(2-[N-(indol-3-yl)méthylamino]éthoxy) éthoxy] méthyl} 4-(2,3-dichlorophényl) 3-éthoxycarbonyl 5-méthoxycarbonyl 6-méthyl 1,4-dihydropyridine et son chlorhydrate sous forme de lyophilisat.

23) d-2-{[2-(2-[N-(quinol-3-yl)méthylamino]éthoxy) éthoxy] méthyl} 4-(2,3-dichlorophényl) 3-éthoxycarbonyl 5-méthoxycarbonyl 6-méthyl 1,4-dihydropyridine et son monoéthyl oxalate PF : 136-138 °C ($CH_3CN$).

Pouvoir rotatoire ([C]$^{21°C}$ = 1 % dans DMSO) :

| λnm | α° |
|---|---|
| 589 | + 29,2 |
| 578 | + 31,2 |
| 546 | + 38,6 |
| 436 | + 128,9 |

24) d-2-{{2-[2-[N-(benzofurazan-5-yl méthyl)amino]éthoxy] éthoxy} méthyl} 4 -(2,3-dichlorophényl) 3-éthoxycarbonyl 5-méthoxycarbonyl 6-méthyl 1,4-dihydropyridine et son monoéthyl oxalate PF : 146-148 °C ($CH_3CN$).

Pouvoir rotatoire ([C]$^{22°C}$ = 1 % dans DMSO) :

| λnm | α° |
|---|---|
| 589 | + 27,5 |
| 578 | + 29,7 |
| 546 | + 37,1 |
| 436 | + 130,5 |

25) d-2-{{2-[2-[N-(benzofurazan-4-yl méthyl)amino]éthoxy] éthoxy} méthyl} 4 -(2,3-dichlorophényl) 3-éthoxycarbonyl 5-méthoxycarbonyl 6-méthyl 1,4-dihydropyridine.

26) d-2-{{2-[2-[N-(benzo-2,1,3-thiadiazol-5-yl méthyl)amino]éthoxy] éthoxy} méthyl} 4-(2,3-dichlorophényl) 3-éthoxycarbonyl 5-méthoxycarbonyl 6-méthyl 1,4-dihydropyridine.

27) d-2-{{2-[2-[N-(benzo-2,1,3-thiadiazol-4-yl méthyl)amino]éthoxy] éthoxy} 4-(2,3-dichlorophényl) 3-éthoxycarbonyl 5-méthoxycarbonyl 6-méthyl 1,4-dihydropyridine.

**Exemple 28**

d-2-{[2-(2-(N-dibenzo(a,d)cyclohept-5-ylamino)éthoxy) éthoxy] méthyl} 4-(2,3-dichlorophényl) 3-éthoxycarbonyl 5-méthoxycarbonyl 6-méthyl 1,4-dihydropyridine

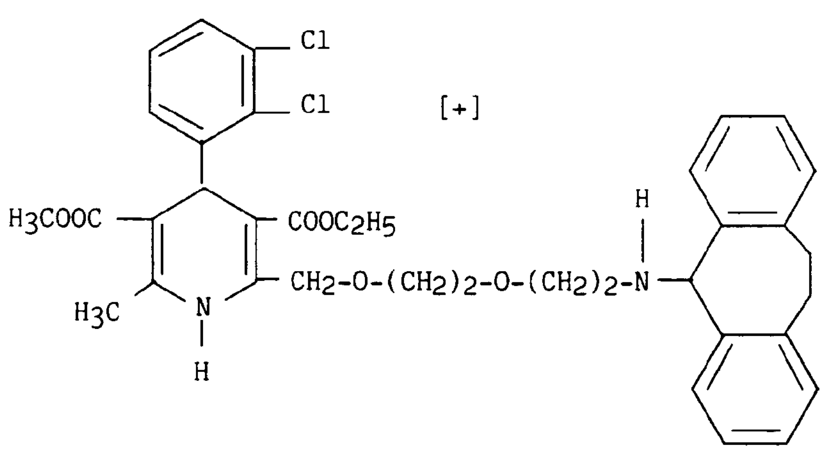

0,01 mole de d-2-{[2-(2-aminoéthoxy) éthoxy] méthyl} 4-(2,3-dichlorophényl) 3-éthoxycarbonyl 5-méthoxycarbonyl 6-méthyl 1,4-dihydropyridine, 0,01 mole de carbonate de potassium, 0,1 g d'iodure de potassium et 0,01 mole de 5-chloro dibenzo(a,d)cycloheptane dans 40 ml d'acétonitrile sont chauffés à reflux sous agitation durant une nuit.

Après dilution à l'eau et extraction, le résidu évaporé est purifié par chromatoflash (éluant : $CHCl_3$ sans alcool/$CH_3COOC_2H_5$, 75/25). On obtient ainsi la d-2-{[2-(2-(N-dibenzo(a,d)cyclohept-5-ylamino)éthoxy) éthoxy] méthyl} 4-(2,3-dichlorophényl) 3-éthoxycarbonyl 5-méthoxycarbonyl 6-méthyl 1,4-dihydropyridine, avec un rendement de 31 %.

0,0029 mole de la base ainsi obtenue est dissoute dans 20 ml d'acétonitrile. On y ajoute 1 ml d'éther chlorhydrique 3 N puis évapore à sec.

On obtient ainsi 1,8 g de chlorhydrate de l'isomère [+] de la 2-{[2-(2-(N-dibenzo(a,d)cyclohept-5-ylamino)éthoxy) éthoxy] méthyl} 4-(2,3-dichlorophényl) 3-éthoxycarbonyl 5-méthoxycarbonyl 6-méthyl 1,4-dihydropyridine PF : 160-162 °C (acétate d'éthyle). Rendement : 86 %.

Pouvoir rotatoire ($[C]^{21°C}$ = 1 % dans DMSO) :

| λnm | α° |
|---|---|
| 589 | + 27,6 |
| 578 | + 29,6 |
| 546 | + 36,7 |

**Exemple 29**

d-2-{{2-[2-[N-méthyl-N-(pyrid-4 yl méthyl)amino]éthoxy] éthoxy} méthyl} 4-(2,3-dichlorophényl) 3-éthoxycarbonyl 5-méthoxycarbonyl 6-méthyl 1,4-dihydropyridine.

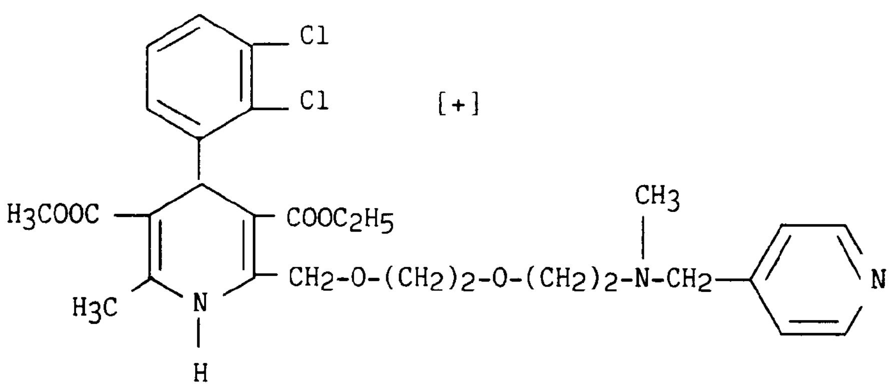

On maintient à 65 °C, sous agitation pendant 4 heures, 0,14 mole de d-2-{{2-[2-[N-pyrid-4 yl méthylamino]éthoxy] éthoxy} méthyl} 4-(2,3-dichlorophényl) 3-éthoxycarbonyl 5-méthoxycarbonyl 6-méthyl 1,4-dihydropyridine avec 0,035 mole de triméthylphosphate. On reprend l'ensemble à l'acétate d'éthyle, lave au bicarbonate de sodium puis à l'eau, épuise à l'acide chlorhydrique normal, basifie à froid les phases acides puis fait une extration à l'acétate d'éthyle.

Le résidu évaporé est soumis à une chromatographie flash ($CH_2Cl_2$-$CH_3OH$) (97-3). On obtient le produit attendu sous forme d'huile. Rendement : 18 %.

La base est salifiée par 2 équivalents d'acide oxalique dans l'éthanol. Après filtration du précipité et recristallisation de l'acétonitrile, on obtient le dioxalate de d-2-{{2-[2-[N-méthyl N-(pyrid-4-yl méthyl)amino]éthoxy] éthoxy} méthyl} 4-(2,3-dichlorophényl 3-éthoxycarbonyl 5-méthoxycarbonyl 6-méthyl 1,4-dihydropyridine, PF : 110-112 °C ($CH_3CN$).

Pouvoir rotatoire ($[C]^{21,6°C}$ = 1 % dans DMSO) :

| λnm | α° |
|---|---|
| 589 | + 24,2 |
| 578 | + 25,5 |
| 546 | + 32,2 |

**Exemple 30**

**Etude pharmacologique**

La résistance aux agents anticancéreux est un obstacle majeur à l'efficacité des drogues antitumorales. Parmi les différents types de résistance, la "Multidrug resistance" (MDR) est particulièrment importante, puisqu'elle est induite par des composés d'origine naturelle, actifs contre les tumeurs solides (Anthracyclines, Vinca alcaloïdes, Epipodophyllotoxines par exemple) et qu'elle est très fréquente dans certains cancers (colon par exemple). Les cellules tumorales, lorsqu'elles sont exposées in vitro ou in vivo à l'une de ces drogues, deviennent résistantes, à divers degrés, à l'ensemble de ces composés. Ce phénomène de résistance est dû à l'action d'une protéine membranaire inductible, la gP 170, dont le rôle est d'augmenter l'efflux du cytotoxique, donc de diminuer sa concentration intracellulaire, d'où la perte de sensibilité de ces cellules à la drogue.

Des médicaments, utilisés dans d'autres pathologies, sont connus pour reverser, partiellement ou totalement, la résistance des cellules tumorales (Tsuruo T., Mechanisms of multidrug resistance and implications for therapy. Int. J. Cancer Res., 79, 285-296, 1988 ; Rothenberg, M. and Ling V., Multidrug resistance : molecular biology and clinical relevance, J.N.C.I., 81, 907-910, 1989 ; Gottesman M.M. and Pastan I., Resistance to multiple chemotherapeutic agents in human cancer cells, Trends Pharmacol. Sci, 9, 54-58, 1989 ; Endicott J.A. and Ling V., The biochemistry of P-glycoprotein-mediated multidrug resistance, Annu. Rev. Biochem., 58, 137-171, 1989.)

L'agent modulateur, lorsqu'il est ajouté en même temps que le cytotoxique, diminue, ou supprime totalement la résistance aux agents antitumoraux. Certains médicaments, comme le vérapamil, l'amiodarone ou la cyclosporine ont été utilisés en clinique pour lever cette résistance, mais leurs propriétés pharmacologiques intrinsèques et leurs toxicités limitent considérablement leur utilisation. D'où l'intérêt de la recherche de composés augmentant la sensibilité des cellules tumorales résistantes, tout en étant dépourvus d'autres propriétés pharmacologiques gênantes et de toxicité. Dans le cas des dihydropyridines, il convient d'augmenter l'activité réversante, et de diminuer les propriétés anticalciques.

De plus, le mécanisme de la résistance à la chloroquine, dévelopée par Plasmodium falciparum, est similaire. Le vérapamil restaure la sensibilité d'une lignée résistante à la chloroquine (Krogstad D.J., Gluzman I.Y., Kule D.E., Oduola A.M.J., Martin S.K., Milhous W.K., Schlessinger P.H., Efflux of Chloroquine from Plasmodium falciparum : mechanism of chloroquine resistance, Science, 238, 1283-1285, 1987 ; Martin S.K., Oduola A.M.J., Milhous W.K., Reversal of Chloroquine resistance in Plasmodium falciparum by Verapamil, Science, 235, 899-901, 1987), ainsi que l'amlodipine, (Deloron P., Basco L.K., Dubois B., Gaudin C., Clavier F., Le Bras J., Verdier F., In vitro and in vivo potentiation of chloroquine against malaria parasites by an enantiomer of amlodipine, Antimicrobial agents and chemotherapy, vol 35, n°7, 1338-1342, 1991) ce qui démontre l'intérêt potentiel de composés réversant la résistance des cellules tumorales pour une utilisation en parasitologie.

L'étude pharmacologique des composés de la présente invention a consisté tout d'abord en un examen in vitro effectué sur des cellules résistantes.

Le paramètre mesuré est la cytotoxicité de la drogue antitumorale, quantifiée en absence et en présence du composé reversant.

On a également mesuré l'effet des produits sur la concentration intracellulaire en adriamycine.

En effet, les composés connus pour réverser la MDR agissent en augmentant la concentration intracellulaire en cytotoxique. Cet effet est la conséquence de l'inhibition de l'action de la gP 170 responsable de l'efflux de la drogue.

Cette étude a été complétée par une étude in vivo, en utilisant une leucémie murine résistante à la vincristine (P 388/VCR) et en associant les produits de l'invention à la vincristine.

Parallèlement a été recherchée l'affinité des produits pour le canal calcique .

**A/ ETUDE SUR DES CELLULES TUMORALES RESISTANTES OU NON**

MATERIEL ET METHODES

1) Activité in vitro

cytotoxicité

Deux lignées cellulaires résistantes ont été utilisées :

- 1 Carcinome épidermoïde humain, KB-A1 dont la résistance a été induite par l'adriamycine (ADR). Son facteur de résistance est de environ 300 par rapport à la lignée sensible (résistance moyenne).
- 1 lignée de poumon de hamster chinois, DC-3F/AD, dont la résistance a été induite par l'actinomycine D. Son facteur de résistance est supérieur à 10 000, c'est donc une lignée extrêmement résistante. Ces deux lignées sont résistantes également aux Vinca alcaloïdes (Vincristine et Vinblastine).

Les cellules sont cultivées dans du milieu de culture (RPMI 1640) complet contenant 10 % de sérum de veau foetal, 2 mM de glutamine ; 50 unités/ml de pénicilline, 50 ug/ml de streptomycine, 10 mM d'Hepes.

Les cellules sont réparties dans des microplaques et exposées aux composés cytotoxiques (Actinomycine D pour la lignée DC-3F/AD et Adriamycine pour la lignée KB-A1) à 9 concentrations (dilutions sériées de 2 en 2). Les produits testés pour leur capacité à réverser la résistance sont ajoutés en même temps que le cytotoxique.

Les cellules sont ensuite incubées pendant 4 jours. Le nombre de cellules viables est ensuite quantifié par un essai colorimétrique, le Microculture Tétrazolium Assay (Carmichael J., DeGraff W.G., Gazdar A.F., Minna J.D. and Mitchell J.R. Evaluation of a tetrazolium-based semiautomated colorimetric assay : assessment of chemosensitivity testing, Cancer Res, 47, 936-942, 1987). Les résultats sont exprimés en $IC_{50}$, concentration en cytotoxique qui inhibe à 50 % la prolifération des cellules traitées par rapport au témoin.

Les résultats sont exprimés en facteur de réversion (FR) avec :

$$FR = \frac{IC_{50} \text{ cytotoxique seul}}{IC_{50} \text{ cytotoxique en présence du composé réversant}}$$

Cytométrie en flux

Certains composés anticancéreux comme l'adriamycine, (ADR) ont la propriété d'être fluorescents après excitation par une source lumineuse de longueur d'onde connue.

Par la mesure de cette fluorescence, il est ainsi possible de mesurer de façon relative la concentraton intracellulaire en ADR. La cytométrie en flux (CMF) est un outil de choix pour effectuer ce type de mesure et ainsi déterminer rapidement si certains composés actifs agissent en augmentant la concentration intracellulaire en ADR.

Les cellules ($5.10^5$ par ml) ont été exposées simultanément à l'ADR à une concentration fixe (50µM) et aux composés testés à différentes concentrations. Après 5 heures d'incubation, l'accumulation de l'ADR intracellulaire a été évalué par CMF. Les analyses ont été réalisées sur un cytomètre en flux ATC3000 (BRUKER-FRANCE) équipé d'un laser argon 2025 (SPECTRA-PHISICS-FRANCE) optimisé à 488 nm pour une puissance de 600 mW.

L'analyse de chacun des échantillons a été effectuée sur un total de 10 000 cellules à une vitesse de 1 000 cellules par seconde.

Les résultats ont été collectés sous forme d'histogrammes linéaires de la fluorescence de l'ADR intra-cellulaire.

Expression des résultats : Pour chacun des histogrammes, le canal moyen (MEAN) de fluorescence a été déterminé par le système informatique de l'appareil. Pour toutes les expériences :

- un contrôle négatif (cellules sans ADR) a fixé le seuil d'autofluorescence.
- un contrôle positif (cellules avec ADR) a déterminé la valeur MEAN = MN1.
- les tubes "tests" (cellules avec ADR et avec produit) ont déterminé pour chacun des produits et à chacune des concentrations les valeurs MEAN = MN2.

Les résultats sont exprimés sous forme de variations de la moyenne de fluorescence obtenue pour chacun des tubes "tests" (MN2) par rapport à la moyenne de fluorescence obtenue avec le contrôle positif (MN1) : VAR-MEAN = MN2-MN1. Le paramètre exprimé est donc l'augmentation de la fluorescence de l'ADR en présence des composés testés.

2) Activité in vivo

Activité antitumorale

La lignée sensible parentale P 388 (leucémie murine) et la sous-lignée résistante à la vincristine, P388/VCR, ont été fournies par le NCI (Frederick, USA). Les cellules tumorales ($10^6$ cellules) ont été inoculées au jour zéro dans la cavité intrapéritonéale à des souris B6D2F1 femelles (Iffa Credo, France) pesant de 18 à 20 g (groupes de 8 à 10 animaux).

Les animaux ont reçu tous les jours pendant 4 jours, à partir du jour 1 :

- une administration de produit de la présente invention à tester à raison de 25 à 150 mg/kg par voie i.p., p.o ou i.v, comme mentionné dans le tableau 4a ;
- 30 à 60 minutes après, une administration de vincristine (pris comme agent anti-tumoral de référence) à raison de 0,25 mg/kg par voie i.p.

L'activité antitumorale est exprimée en :

$$\frac{T}{C}\% = \frac{\text{Temps de survie médian des animaux traités}}{\text{Temps de survie médian des animaux contrôles}} \times 100$$

Les valeurs sont des moyennes obtenues dans des expériences indépendantes (± sem quand n est supérieur ou égal à 3).

RESULTATS

1) Activité in vitro

Cytotoxicité

Le tableau 1 donne les valeurs des facteurs de réversions obtenus avec les différents composés avec la lignée DC-3F/AD et le tableau 2 avec la lignée KB-A1.

Tous les produits testés relatifs aux exemples de la présente invention sont beaucoup plus actifs que les produits de référence, et certains d'entre eux réversent totalement la résistance des cellules KB-A1.

Cytométrie en flux

Le tableau 3 donne l'augmentation de la fluorescence de l'ADR (VAR-MEAN) obtenue avec les différents composés sur la lignée KB-A1.

Tous les produits des exemples de l'invention testés sont plus actifs que les produits de référence.

2) Activité in vivo

Les tableaux 4a et 4b montrent l'augmentation de l'activité antitumorale in vivo de la vincristine obtenue avec différents composés représentatifs de la présente invention.

Tous les produits des exemples de l'invention testés augmentent considérablement l'activité antitumorale de la vincristine dans des cellules résistantes, en restaurant une sensibilité voisine de celle des cellules sensibles.

Sur la lignée sensible (P388) les produits de l'invention testés accroissent notablement la sensibilité des cellules à la vincristine.

**TABLEAU 1**

| FACTEURS DE REVERSION AVEC LA LIGNEE DC-3F/AD | | | |
|---|---|---|---|
| **PRODUITS** | **1** µM | **2,5** µM | **5** µM |
| Produits de reférence | | | |
| VERAPAMIL | 1,0 | 1,5 | 3,1 |
| NIFEDIPINE | 1,2 | 2,3 | 5,8 |
| AMLODIPINE | 1,0 | 1,4 | 2,6 |
| NK 250 | 1,1 | 1,3 | 2,0 |
| Produits des exemples | | | |
| 1 | 21,0 | 1 042,7 | 2 613,2 |
| 2 | 1,5 | 141,3 | 1 478,2 |
| 4 | 23,0 | 294,4 | 3 962,3 |
| 5 | 7,6 | 267,2 | 3 567,0 |
| 6 | 9,6 | 599,8 | 4 340,9 |
| 8 | 11,4 | 195,6 | 1 319,9 |
| 9 | 1,2 | 53,9 | 560,3 |
| 11 | 1,0 | 394,8 | 2 442,4 |
| 12 | 1,1 | 86,1 | 1 320,1 |
| 13 | 19,4 | 622,7 | 4 141,7 |
| 14 | 8,2 | 482,7 | 4 463,2 |
| 15 | 301,4 | 4 375,5 | 6 510,3 |
| 16 | 3,4 | 445,1 | 8 248,4 |
| 17 | 3,7 | 706,1 | 8 600,0 |
| 18 | 3,3 | 1 082,1 | 5 018,4 |
| 19 | 1,5 | 305,7 | 1 701,7 |
| 23 | 10,2 | 310,0 | 2 304,0 |
| 28 | 1,4 | 21,5 | 447,3 |

**TABLEAU 2**

| FACTEURS DE REVERSION AVEC LA LIGNEE KB-A1 | | | |
|---|---|---|---|
| **PRODUITS** | **1** µM | **2,5** µM | **5** µM |
| Produits de reférence | | | |
| VERAPAMIL | 4,6 | 16,7 | 26,0 |
| NIFEDIPINE | 1,0 | 1,0 | 1,0 |
| AMLODIPINE | 1,5 | 2,4 | 5,9 |
| NK 250 | 0,8 | 2,3 | 3,1 |
| Produits des exemples | | | |
| 1 | 32,1 | 210,6 | 640,5 |
| 2 | 2,2 | 90,6 | 389,5 |
| 4 | 26,0 | 106,4 | 473,7 |
| 5 | 13,2 | 96,5 | 225,8 |
| 6 | 15,2 | 54,7 | 159,1 |
| 8 | 6,9 | 28,2 | 136,0 |
| 9 | 2,4 | 41,8 | 99,0 |
| 11 | 1,7 | 126,3 | 336,0 |
| 12 | 1,2 | 47,0 | 125,4 |
| 13 | 12,3 | 66,8 | 165,1 |
| 14 | 22,7 | 88,0 | 169,4 |
| 15 | 12,1 | 65,3 | 281,9 |
| 16 | 13,9 | 49,2 | 122,4 |
| 17 | 10,5 | 45,7 | 120,7 |
| 18 | 4,3 | 193,3 | 348,2 |
| 19 | 3,6 | 40,3 | 140,5 |
| 23 | 19,6 | 101,2 | 1035,8 |

**TABLEAU 3**

| MESURE DE L'ACCUMULATION INTRACELLULAIRE EN ADR LIGNEE KB-A1 | | | |
|---|---|---|---|
| **PRODUITS** | **CONCENTRATION (μM)** | | |
| | **1** | **2,5** | **5** |
| Produits de référence | | | |
| VERAPAMIL | 3,7 | 8,6 | 12,2 |
| NIFEDIPINE | 0,0 | 0,0 | 0,0 |
| AMLODIPINE | 0,0 | 2,1 | 4,7 |
| Produits des exemples | | | |
| 1 | 5,5 | 26,1 | 45,7 |
| 4 | 2,0 | 7,0 | 18,6 |
| 8 | 2,0 | 9,4 | 24,2 |
| 11 | 0,2 | 18,2 | 31,0 |
| 14 | 2,4 | 8,2 | 20,5 |
| 15 | 7,0 | 16,1 | 38,6 |
| 19 | 9,4 | 15,7 | 34,7 |

**TABLEAU 4a**

| AUGMENTATION DE L'ACTIVITE ANTITUMORALE DE LA VINCRISTINE PAR LES PRODUITS DE LA PRESENTE INVENTION SUR LA LIGNEE RESISTANTE P 388/VCR | | | | |
|---|---|---|---|---|
| **VCR i.p.** | **PRODUITS DES EXEMPLES** | | | **T/C %** |
| | **Exemples** | **Dose** | **Voie** | |
| 0,25 mg/kg | - | - | - | 150 |
| 0 | 1 | 50 mg/kg | i.p | 107 |
| 0 | 1 | 150 mg/kg | p.o | 99 |
| 0,25 mg/kg | 1 | 50 mg/kg | i.p | 171 |
| 0,25 mg/kg | 1 | 150 mg/kg | p.o | 185 |
| 0 | 4 | 50 mg/kg | i.p | 100 |
| 0 | 4 | 150 mg/kg | p.o | 110 |
| 0,25 mg/kg | 4 | 50 mg/kg | i.p | 176 |
| 0,25 mg/kg | 4 | 150 mg/kg | p.o | 181 |
| 0 | 11 | 50 mg/kg | i.p | 100 |

| | | | | |
|---|---|---|---|---|
| 0 | 11 | 150 mg/kg | p.o | 99 |
| 0,25 mg/kg | 11 | 50 mg/kg | i.p | 146 |
| 0,25 mg/kg | 11 | 150 mg/kg | p.o | 177 |
| | | | | |
| 0 | 14 | 50 mg/kg | i.p | 99 |
| 0 | 14 | 150 mg/kg | p.o | 103 |
| 0,25 mg/kg | 14 | 50 mg/kg | i.p | 164 |
| 0,25 mg/kg | 14 | 150 mg/kg | p.o | 200 |
| | | | | |
| 0 | 15 | 25 mg/kg | i.p | 109 |
| 0 | 15 | 25 mg/kg | p.o | 112 |
| 0 | 15 | 25 mg/kg | i.v | 94 |
| 0,25 mg/kg | 15 | 25 mg/kg | i.p | 262 |
| 0,25 mg/kg | 15 | 25 mg/kg | p.o | 180 |
| 0,25 mg/kg | 15 | 25 mg/kg | i.v | 186 |
| | | | | |
| 0 | 28 | 50 mg/kg | i.p | 107 |
| 0 | 28 | 150 mg/kg | p.o | 99 |
| 0,25 mg/kg | 28 | 50 mg/kg | i.p | 180 |
| 0,25 mg/kg | 28 | 150 mg/kg | p.o | 207 |

TABLEAU 4b

| AUGMENTATION DE L'ACTIVITE ANTITUMORALE DE LA VINCRISTINE PAR LES PRODUITS DE LA PRESENTE INVENTION SUR LA LIGNEE SENSIBLE P388 | | | | | |
|---|---|---|---|---|---|
| **VCR i.p.** | **PRODUITS DES EXEMPLES** | | | **T/C %** | **Survivants à J 60** |
| | **Exemples** | **Dose** | **Voie** | | |
| 0,25 mg/kg | - | - | - | 239 | 0/8 |
| 0 | 1 | 150 mg/kg | p.o | 99 | 0/8 |
| 0,25 mg/kg | 1 | 150 mg/kg | p.o | >532 | 4/8 |
| 0 | 8 | 150 mg/kg | p.o | 106 | 0/8 |
| 0,25 mg/kg | 8 | 150 mg/kg | p.o | 294 | 2/8 |
| 0 | 14 | 150 mg/kg | p.o | 95 | 0/8 |
| 0,25 mg/kg | 14 | 150 mg/kg | p.o | >587 | 4/8 |
| 0 | 15 | 12,5 mg/kg | p.o | 101 | 0/8 |
| 0,25 mg/kg | 15 | 12,5 mg/kg | p.o | 540 | 3/8 |

**B/ AFFINITE DES PRODUITS DE L'INVENTION POUR LE CANAL CALCIQUE MARQUE PAR LE [$^3$H] PN 200110**

Les essais ont été réalisés avec des préparations de microsomes de muscle lisse d'aorte de porc selon les conditions expérimentales décrites par A. GOLL (FEBS Lett. 1983, 157, 63-9).

Les résultats sont exprimés en $IC_{50}$ (M) et représentent des moyennes de trois expériences indépendantes. Ils sont regroupés dans le tableau 5.

TABLEAU 5

| PRODUITS | $IC_{50}$(M) |
|---|---|
| Exemple 1 | $1,6.10^{-6}$ |
| 12 | $1,3.10^{-6}$ |
| 14 | $1,0.10^{-6}$ |
| 16 | $1,6.10^{-6}$ |
| 17 | $1,4.10^{-6}$ |
| 18 | $1,1.10^{-6}$ |
| 19 | $3,2.10^{-6}$ |
| 20 | $2,1.10^{-6}$ |
| 22 | $3,3.10^{-6}$ |
| 23 | $2,0.10^{-6}$ |
| NIFEDIPINE | $2,5.10^{-8}$ |

Ces résultats montrent que, contrairement à la Nifédipine, les produits de l'invention ne possèdent que peu d'affinité sur le canal calcique et se présentent donc comme de très faibles agents anticalciques.

RESUME

L'ensemble des résultats regroupés dans les tableaux 1 à 5 montre que les composés de la présente invention :

- ont une faible activité anticalcique, 100 fois plus faible que celle du produit de référence,
- sont peu toxiques, même administrés à de fortes doses pendant 4 jours chez la souris,
- augmentent considérablement, in vitro, la sensibilité des cellules résistantes aux agents antitumoraux, y compris dans le cas de cellules présentant le phénotype MDR, et à des concentration de l'ordre du micromolaire,
- augmentent de façon importante l'accumulation d'adriamycine dans les cellules résistantes,
- augmentent de façon importante la survie des animaux porteurs d'une leucémie résistante (P388/VCR) (quand ils sont administrés en même temps que la vincristine).
- augmentent l'efficacité, in vivo, des agents antitumoraux sur les cellules sensibles, ce qui se traduit par l'apparition d'un nombre élévé de survivants dans les groupes traités. Ceci a pour conséquence la possibilité de réduire les doses des médicaments cytotoxiques utilisés dans l'association.

CONCLUSION

D'après l'étude pharmacologique effectuée, les composés de la présente invention accroissent notablement la sensibilité des cellules tumorales aux agents antitumoraux, que ces cellules aient ou non acquis une résistance à différents agents antitumoraux, tout en ne présentant que de très faibles propriétés anticalciques et par voie de conséquence, n'ayant pas d'effet hypotenseur. Ceci permet donc l'utilisation des dits produits en therapeutique et notamment en thérapeutique anticancéreuse sans entrainer d'effets secondaires gênants.

## Revendications

**1-** Les composés de 1,4-dihydropyridine de formule I :

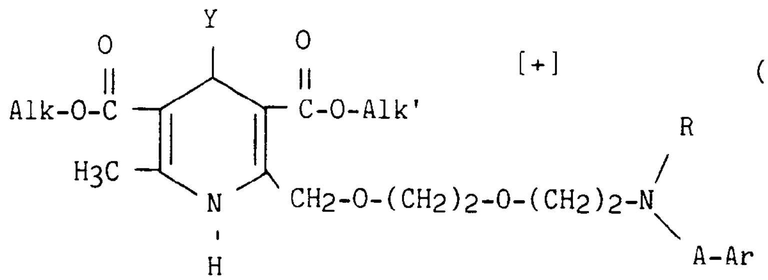

(I)

dans laquelle:

- Y représente un radical phényle éventuellement substitué par 1 à 5 substituants identiques ou différents représentant chacun un atome d'halogène, ou un radical alkoxy ou alkylthio ayant chacun de 1 à 4 atomes de carbone, un radical trihalogénométhyle ou un radical méthylène-dioxy ;
- Alk et Alk', identiques ou différents, représentent chacun un radical alkyle de 1 à 5 atomes de carbone, en chaine linéaire ou ramifiée ;
- R représente un atome d'hydrogène, un radical alkyle de 1 à 5 atomes de carbone en chaine linéaire ou ramifiée, ou un radical alkényle ou alkinyle ayant chacun de 3 à 5 atomes de carbone en chaine linéaire ou ramifiée ;
- A représente :
  une chaine $-(CH_2)_m-$ dans laquelle m est un nombre entier de 1 à 5, éventuellement substituée par un radical alkyle de 1 à 5 atomes de carbone en chaine droite ou ramifiée, ou par un radical alkényle ou alkinyle ayant de 2 à 5 atomes de carbone en chaine droite ou ramifiée,

et simultanément

- Ar représente :
  a) un radical phényle éventuellement substitué par un ou plusieurs atomes d'halogène ou radicaux trifluorométhyle, nitro, cyano, alkoxycarbonyl de 2 à 6 atomes de carbone ou alkylthio de 1 à 5 atomes de carbone,
  b) un radical thiényle, pyridyle, naphtyle, quinolyle, isoquinolyle, indolyle, N-méthylindolyle, benzofurazanyle ou benzo-2,1,3-thiadiazolyle,
  ou
- l'ensemble -A-Ar représente un radical dibenzo(a,d)cyclohept-5-yle ; sous forme d'isomère dextrogyre.

**2-** Les sels d'addition physiologiquement tolérables des composés de la revendication 1 avec des acides appropriés.

**3-** La d-2-{(2-(2-(N-p-nitrobenzylamino)éthoxy)éthoxy] méthyl} 4-(2,3-dichlorophényl) 3-éthoxycarbonyl 5-méthoxycarbonyl 6-méthyl 1,4-dihydropyridine, son monoéthyl oxalate et son chlorhydrate.

**4-** La d-2-{(2-(2-(N-p-cyanobenzylamino)éthoxy)éthoxy] méthyl} 4-(2,3-dichlorophényl) 3-éthoxycarbonyl 5-méthoxycarbonyl 6-méthyl 1,4-dihydropyridine, et son monoéthyl oxalate.

**5-** La d-2-{[2-(2-(N-thién-3-ylméthylamino)éthoxy)éthoxy] méthyl} 4-(2,3-dichlorophényl) 3-éthoxycarbonyl 5-méthoxycarbonyl 6-méthyl 1,4-dihydropyridine, son monoéthyl oxalate et son fumarate.

**6-** La d-2-{[2-(2-(N-pyrid-4-ylméthylamino)éthoxy)éthoxy] méthyl} 4-(2,3-dichlorophényl) 3-éthoxycarbonyl 5-méthoxycarbonyl 6-méthyl 1,4-dihydropyridine, et son monoéthyl oxalate.

**7-** La d-2-{[2-(2-(N-quinol-4-ylméthylamino)éthoxy)éthoxy] méthyl} 4-(2,3-dichlorophényl) 3-éthoxycarbonyl 5-méthoxycarbonyl 6-méthyl 1,4-dihydropyridine, et son monoéthyl oxalate.

**8-** La d-2-{[2-(2-(N-p-fluorobenzylamino)éthoxy)éthoxy] méthyl} 4-(2,3-dichlorophényl) 3-éthoxycarbonyl 5-méthoxycarbonyl 6-méthyl 1,4-dihydropyridine, et son monoéthyl oxalate.

**9-** La d-2-{[2-(2-(N-napht-1-yl méthylamino)éthoxy)éthoxy] méthyl} 4-(2,3-dichlorophényl) 3-éthoxycarbonyl 5-méthoxycarbonyl 6-méthyl 1,4-dihydropyridine, et son monoéthyl oxalate.

**10-** La d-2-{[2-(2-(N-o-nitrobenzylamino)éthoxy)éthoxy] méthyl} 4-(2,3-dichlorophényl) 3-éthoxycarbonyl 5-méthoxycarbonyl 6-méthyl 1,4-dihydropyridine et son monoéthyl oxalate.

**11-** La d-2-{{2-[2-[N-(benzofurazan-5-yl méthyl)amino]éthoxy] éthoxy} méthyl} 4-(2,3-dichlorophényl) 3-éthoxycarbonyl 5-méthoxycarbonyl 6-méthyl 1,4-dihydropyridine et son monoéthyl oxalate.

**12-** La d-2-{{2-[2-[N-méthyl-N-(pyrid-4-yl méthyl)amino]éthoxy] éthoxy} méthyl} 4-(2,3-dichlorophényl) 3-éthoxycarbonyl 5-méthoxycarbonyl 6-méthyl 1,4-dihydropyridine et son dioxalate.

**13-** Le procédé de préparation des composés de la revendication 1, caractérisé en ce que l'on transforme l'isomère dextrogyre de l'amine primaire de formule II :

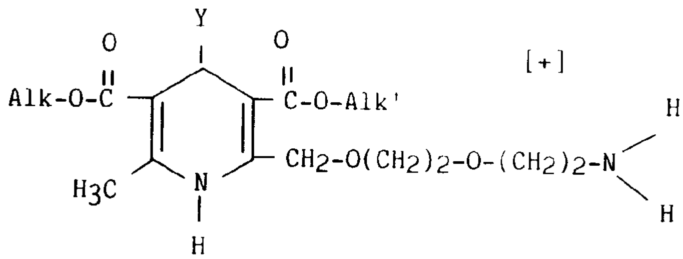

(II)

(dans laquelle Y, Alk et Alk' ont les significations définies dans la revendication 1) en isomère dextrogyre de l'amine secondaire ou tertiaire correspondant de formule I telle que définie dans la revendication 1.

**14-** Le procédé selon la revendication 13 pour préparer l'isomère dextrogyre des composés I répondant plus précisémment à la formule Ia :

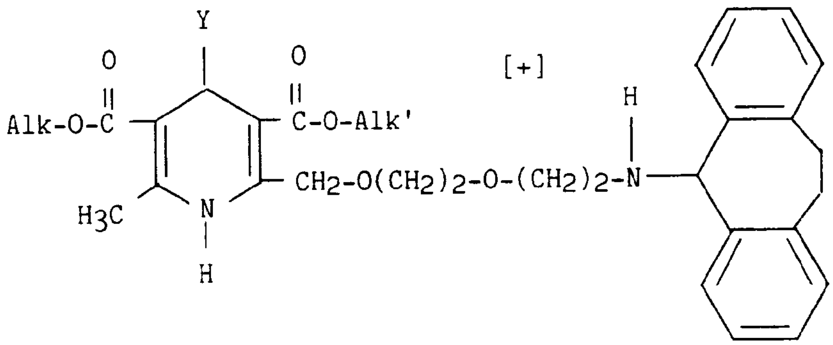

(Ia)

dans laquelle Y, Alk et Alk' ont les significations définies dans la revendication 1, caractérisé en ce que l'on traite :

- l'isomère dextrogyre de l'amine primaire de formule II définie dans la revendication 12,
- par un agent de formule IIIa :

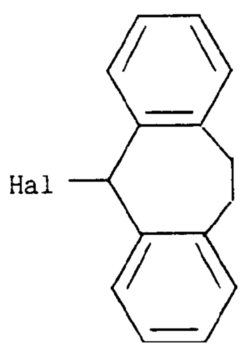

(IIIa)

dans laquelle Hal représente un atome d'halogène.

**15-** Le procédé selon la revendication 13 pour préparer l'isomère dextrogyre des composés I répondant plus précisémment à la formule Ib :

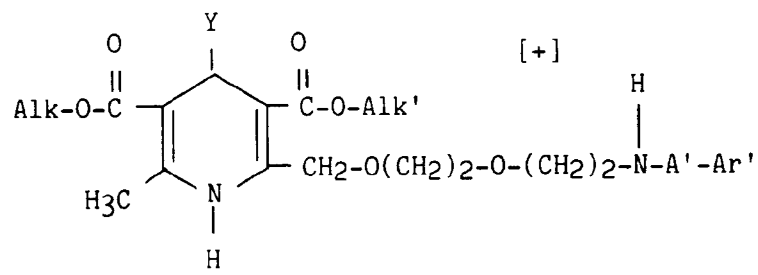

(Ib)

[dans laquelle Y, Alk et Alk' ont les significations définies dans la revendication 1 et

- A' représente :

  une chaine $(CH_2)_m$ dans laquelle m est un nombre entier de 1 à 5, éventuellement substituée par un radical alkyle ayant de 1 à 5 atomes de carbone en chaine droite ou ramifiée, ou par un radical alkényle ou alkynyle ayant chacun de 2 à 5 atomes de carbone en chaine droite ou ramifiée,

  et simultanément

- Ar' représente :

  a) un radical phényle éventuellemeni substitué par un ou plusieurs atomes d'halogène ou radicaux trifluorométhyle, nitro, cyano, alkoxycarbonyle de 2 à 6 atomes de carbone, ou alkylthio de 1 à 5 atomes de carbone, ou

  b) un radical thiényle, pyridyle, naphtyle, quinolyle, isoquinolyle, indolyle, N-méthyindolyle, benzofurazanyle ou benzo-2,1,3-thiadiazolyle],

  caractérisé en ce que : l'on traite l'isomère dextrogyre de l'amine primaire de formule II définie dans la revendication 13 :

  par un agent de formule III b :

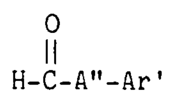

(III b)

dans laquelle :

- Ar' a la signification précédemment définie,
- A" représente une chaîne $(CH_2)_{m-1}$ dans laquelle m est un nombre entier de 1 à 5, éventuellement substituée par un radical alkyle ayant de 1 à 5 atomes de carbone en chaine droite ou ramifiée, ou par un radical alkényle ou alkynyle ayant chacun de 2 à 5 atomes de carbone en chaine droite ou ramifiée,

la réaction s'effectuant dans un solvant approprié en présence de $NaBH_4$.

**16-** Le procédé selon la revendication 13 pour préparer l'isomère dextrogyre des composés I répondant plus précisémment aux formules Ic et Id :

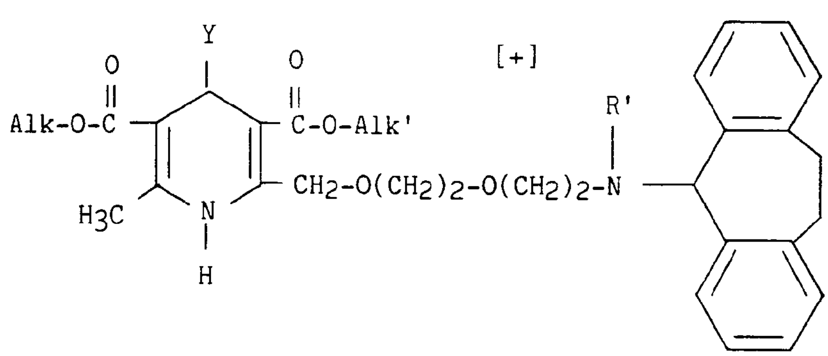

(Ic)

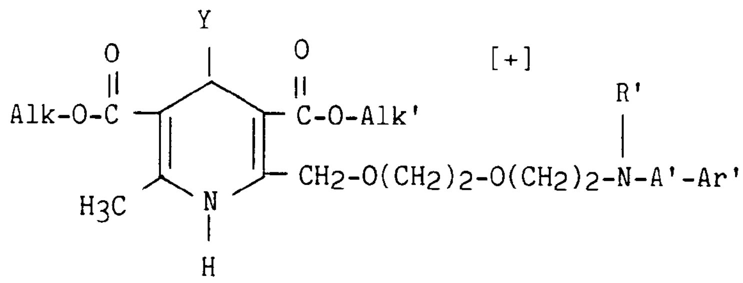

(Id)

dans chacune de ces formules :

- Y, Alk, Alk', A' et Ar' ont les significations définies dans la revendication 13, et
- R' représente un radical alkyle de 1 à 5 atomes de carbone en chaine droite ou ramifiée, ou un radical alkényle ou alkinyle ayant chacun de 3 à 5 atomes de carbone en chaine linéaire ou ramifiée (c'est à dire R' a la même signification que R à l'exception d'un atome d'hydrogène),

caractérisé en ce que :
l'on alcoyle les composés Ia et Ib définis respectivement dans les revendications 14 et 15,
soit en condensant les composés Ia et Ib avec un agent de formule IV :

R"-CO-Cl (IV)

dans laquelle :
R" représente un radical alkyle de 1 à 4 atomes de carbone en chaine linéaire ou ramifiée, ou un radical alkényle ou alkinyle ayant chacun de 2 à 4 atomes de carbone en chaine linéaire ou ramifiée ;
puis en réduisant l'amide ainsi obtenue par $LiAlH_4$,
pour obtenir les composés de formule $Ic_1$ et $Id_1$ :

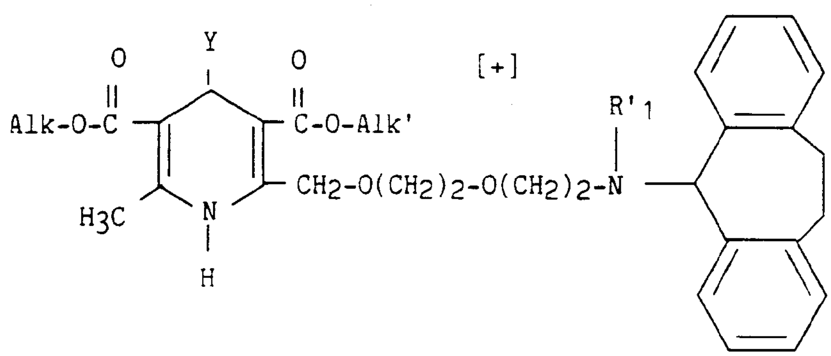

($Ic_1$)

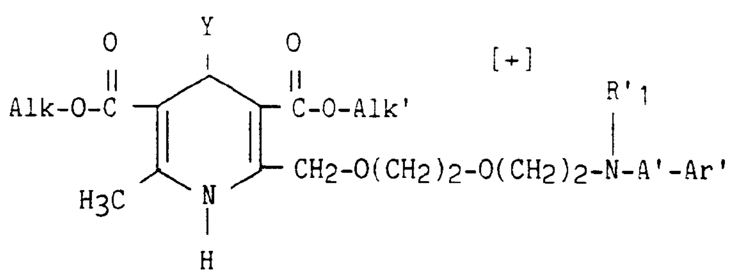

($Id_1$)

dans lesquelles:

- Y, Alk, Alk', A' et Ar' ont les significations précédemment définies et
- $R'_1$ représente un radical alkyle de 2 à 5 atomes de carbone en chaine droite ou ramifiée ou un radical alkényle ou alkinyle ayant chacun de 3 à 5 atomes de carbone en chaine linéaire ou ramifiée ;
  - soit en faisant réagir les composés Ia et Ib avec acide formique et formol ou phosphate de méthyle à une température comprise entre 60 et 100 °C, pour obtenir les composés de formule $Ic_2$ et $Id_2$.

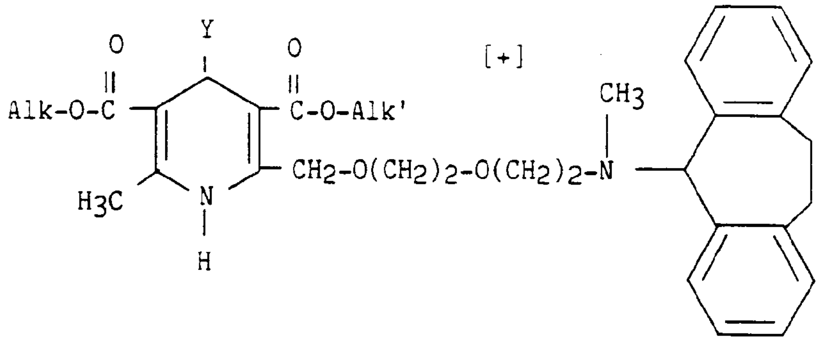

(Ic2)

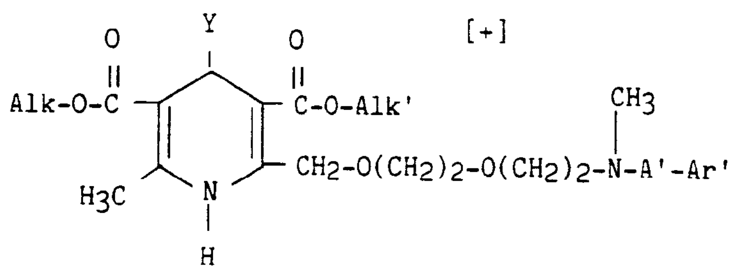

(Id2)

dans lesquelles :

- Y, Alk, Alk', A' et Ar' ont les significations précédemment définies.

**17-** Les compositions pharmaceutiques contenant comme principe actif un composé selon les revendications 1 à 12 avec des excipients pharmaceutiques appropriés.

**18-** Les compositions pharmaceutiques selon la revendication 17 ayant la propriété de potentialiser l'activité des cytotoxiques et d'augmenter la sensibilité des cellules tumorales aux agents antitumoraux sans entrainer d'effets secondaires gênants.

**Revendications pour l'Etat contractant suivant : ES**

**1-** Le procédé de préparation des composés de 1,4-dihydropyridine de formule I :

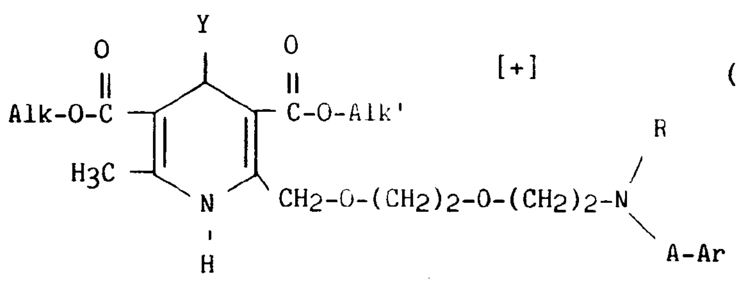

(I)

dans laquelle :

- Y représente un radical phényle éventuellement substitué par 1 à 5 substituants identiques ou différents représentant chacun un atome d'halogène, ou un radical alkoxy ou alkylthio ayant chacun de 1 à 4 atomes de carbone, un radical trihalogénométhyle ou un radical méthylène-dioxy ;
- Alk et Alk', identiques ou différents, représentent chacun un radical alkyle de 1 à 5 atomes de carbone, en chaine linéaire ou ramifiée ;
- R représente un atome d'hydrogène, un radical alkyle de 1 à 5 atomes de carbone en chaine linéaire ou ramifiée, ou un radical alkényle ou alkinyle ayant chacun de 3 à 5 atomes de carbone en chaine linéaire ou ramifiée ;
- A représente :

  une chaine $-(CH_2)_m-$ dans laquelle m est un nombre entier de 1 à 5, éventuellement substituée par un radical alkyle de 1 à 5 atomes de carbone en chaine droite ou ramifiée, ou par un radical alkényle ou alkinyle ayant de 2 à 5 atomes de carbone en chaine droite ou ramifiée,

et simultanément

- Ar représente :

  a) un radical phényle éventuellement substitué par un ou plusieurs atomes d'halogène ou radicaux trifluorométhyle, nitro, cyano, alkoxycarbonyl de 2 à 6 atomes de carbone ou alkylthio de 1 à 5 atomes de carbone,

  b) un radical thiényle, pyridyle, naphtyle, quinolyle, isoquinolyle, indolyle, N-méthylindolyle, benzofurazanyle ou benzo-2,1,3-thiadiazolyle,

  ou

- l'ensemble -A-Ar représente un radical dibenzo(a,d)cyclohept-5-yle ; sous forme d'isomère dextrogyre, et leurs sels d'addition physiologiquement tolérables avec des acides appropriés,
- caractérisé en ce que l'on transforme l'isomère dextrogyre de l'amine primaire de formule II :

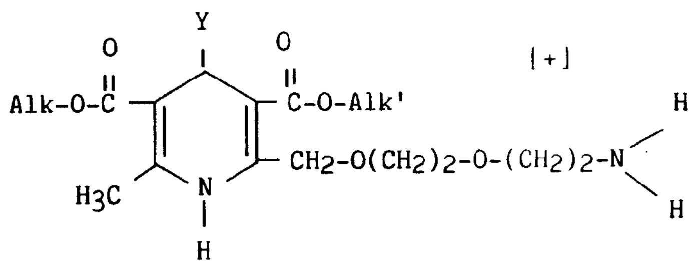

(II)

(dans laquelle Y, Alk et Alk' ont les significations précedemment définies)

en isomère dextrogyre de l'amine secondaire ou tertiaire correspondant de formule I telle que définie précédemment,

et si on le désire, on salifie l'amine obtenue avec des acides appropriés.

**2-** Le procédé selon la revendication 1 pour préparer l'isomère dextrogyre des composés I répondant plus précisémment à la formule Ia :

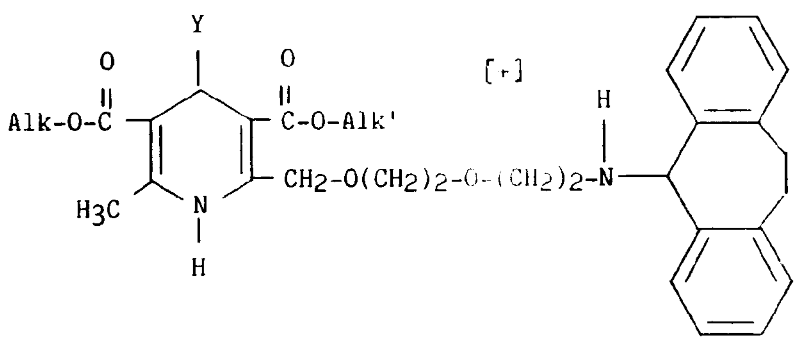

(Ia)

dans laquelle Y, Alk et Alk' ont les significations définies dans la revendication 1,

caractérisé en ce que l'on traite :

- l'isomère dextrogyre de l'amine primaire de formule II définie dans la revendication 1,
- par un agent de formule IIIa :

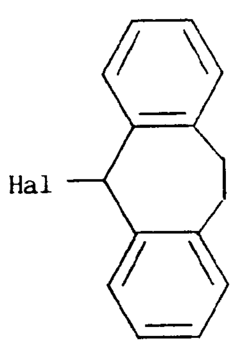

(IIIa)

dans laquelle Hal représente un atome d'halogène,
et si on le désire, on salifie l'amine obtenue avec des acides appropriés.

**3-** Le procédé selon la revendication 1 pour préparer l'isomère dextrogyre des composés I répondant plus précisément à la formule Ib :

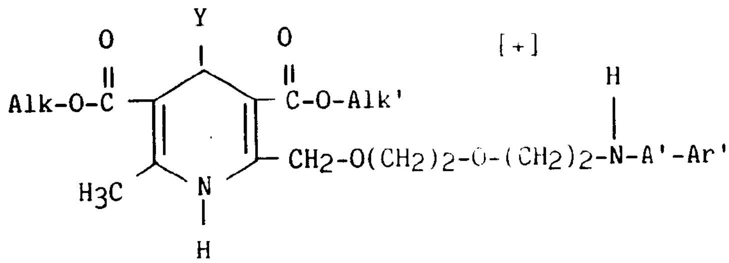
(Ib)

[dans laquelle Y, Alk et Alk' ont les significations définies dans la revendication 1 et

- A' représente :
  une chaine $(CH_2)_m$ dans laquelle m est un nombre entier de 1 à 5, éventuellement substituée par un radical alkyle ayant de 1 à 5 atomes de carbone en chaine droite ou ramifiée, ou par un radical alkényle ou alkynyle ayant chacun de 2 à 5 atomes de carbone en chaine droite ou ramifiée,
  et simultanément
- Ar' représente :
  a) un radical phényle éventuellement substitué par un ou plusieurs atomes d'halogène ou radicaux trifluorométhyle, nitro, cyano, alkoxycarbonyle de 2 à 6 atomes de carbone, ou alkylthio de 1 à 5 atomes de carbone, ou
  b) un radical thiényle, pyridyle, naphtyle, quinolyle, isoquinolyle, indolyle, N-méthyindolyle, benzofurazanyle ou benzo-2,1,3-thiadiazolyle],

caractérisé en ce que : l'on traite l'isomère dextrogyre de l'amine primaire de formule II définie dans la revendication 1 :

- par un agent de formule III b :

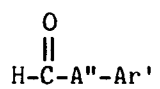
(III b)

dans laquelle :

- Ar' a la signification précédemment définie et
- A" représente une chaîne $(CH_2)_{m-1}$ dans laquelle m est un nombre entier de 1 à 5, éventuellement substituée par un radical alkyle ayant de 1 à 5 atomes de carbone en chaine droite ou ramifiée, ou par un radical alkényle ou alkynyle ayant chacun de 2 à 5 atomes de carbone en chaine droite ou ramifiée,

la réaction s'effectuant dans un solvant approprié en présence de $NaBH_4$,
et si on le désire, on salifie l'amine obtenue avec des acides appropriés.

**4-** Le procédé selon la revendication 1 pour préparer l'isomère dextrogyre des composés I répondant plus précisément aux formules Ic et Id :

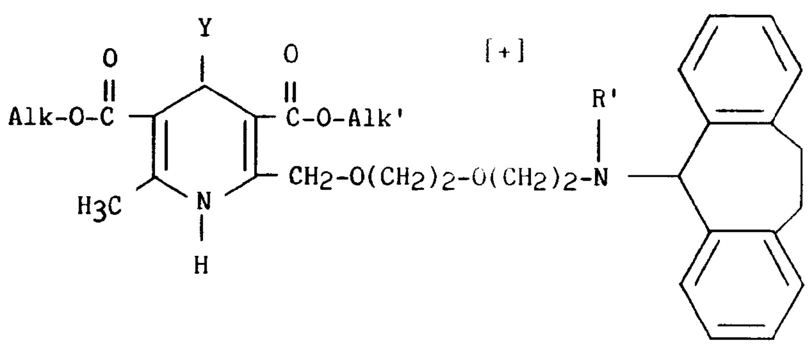
(Ic)

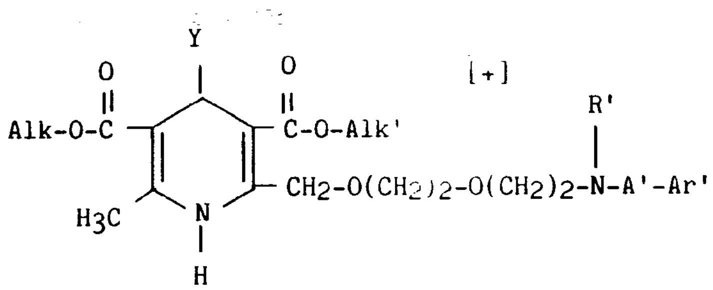

(Id)

dans chacune de ces formules :

- Y, Alk, Alk', A' et Ar' ont les significations définies dans la revendication 3, et
- R' représente un radical alkyle de 1 à 5 atomes de carbone en chaine droite ou ramifiée, ou un radical alkényle ou alkinyle ayant chacun de 3 à 5 atomes de carbone en chaine linéaire ou ramifiée (c'est à dire R' a la même signification que R à l'exception d'un atome d'hydrogène),

caractérisé en ce que :

l'on alcoyle les composés Ia et Ib définis respectivement dans les revendications 2 et 3,

- soit en condensant les composés Ia et Ib avec un agent de formule IV :

R"-CO-Cl (IV)

dans laquelle :

- R" représente un radical alkyle de 1 à 4 atomes de carbone en chaine linéaire ou ramifiée, ou un radical alkényle ou alkinyle ayant chacun de 2 à 4 atomes de carbone en chaine linéaire ou ramifiée ;
  puis en réduisant l'amide ainsi obtenue par $LiAlH_4$,
  pour obtenir les composés de formule $Ic_1$ et $Id_1$ :

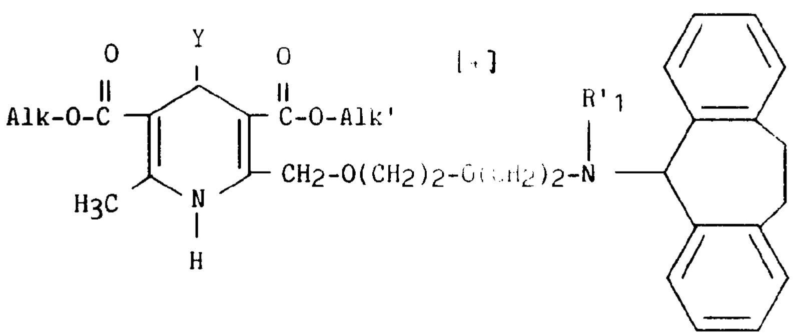

($Ic_1$)

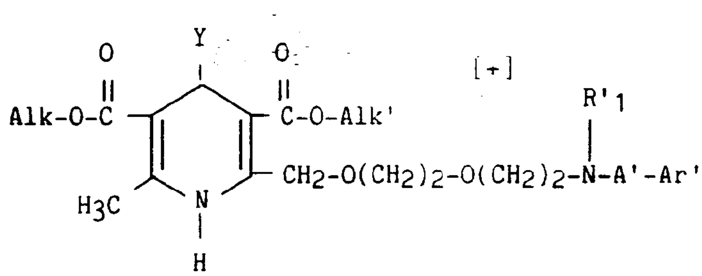

($Id_1$)

dans lesquelles :

- Y, Alk, Alk', A' et Ar' ont les significations précédemment définies et
- $R'_1$ représente un radical alkyle de 2 à 5 atomes de carbone en chaine droite ou ramifiée ou un radical alkényle ou alkinyle ayant chacun de 3 à 5 atomes de carbone en chaine linéaire ou ramifiée ;

- soit en faisant réagir les composés Ia et Ib avec acide formique et formol ou phosphate de méthyle à une température comprise entre 60 et 100 °C, pour obtenir les composés de formule $Ic_2$ et $Id_2$.

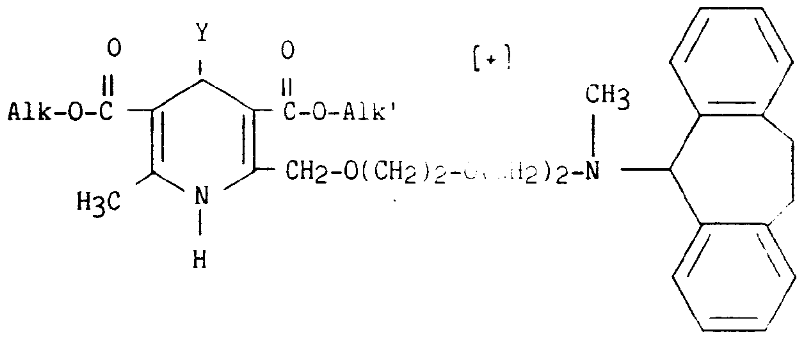

(Ic2)

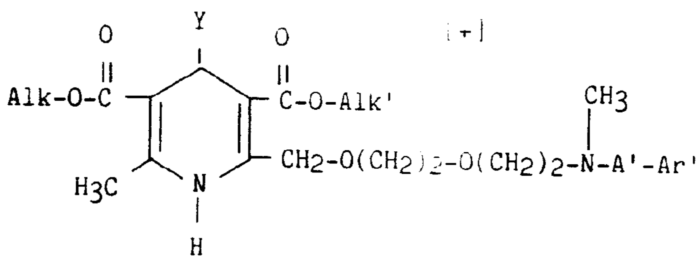

(Id2)

dans lesquelles :

- Y, Alk, Alk', A' et Ar' ont les significations précédemment définies,

et si on le désire, on salifie l'amine obtenue avec des acides appropriés.

Office européen des brevets

# RAPPORT DE RECHERCHE EUROPEENNE

Numero de la demande
EP 93 40 0272

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A,D | EP-A-0 259 206 (ADIR ET CIE)<br>* page 6, ligne 40 - page 7, ligne 4; revendications 1,2,11-13; exemples 7,11 * | 1,2,17, 18 | C07D211/90<br>C07D409/12<br>C07D401/12<br>C07D405/04<br>C07D409/14<br>C07D405/14<br>A61K31/44<br>A61K31/47 |
| A,D | EP-A-0 406 502 (ADIR ET CIE)<br>* page 5, ligne 56 - page 6, ligne 21; revendications 1,3-6 * | 1,2,17, 18 | |
| A | WO-A-9 118 599 (BYK GULDEN LOMBERG CHEMISCHE FABRIK GMBH) | | |
| | | | DOMAINES TECHNIQUES RECHERCHES (Int. Cl.5) |
| | | | C07D<br>A61K |

Le présent rapport a été établi pour toutes les revendications

| Lieu de la recherche | Date d'achèvement de la recherche | Examinateur |
|---|---|---|
| BERLIN | 08 AVRIL 1993 | VAN AMSTERDAM L. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)